# EUROPEAN PATENT SPECIFICATION

(11) **EP 0 522 075 B1**
(45) Date of publication and mention of the grant of the patent: **29.05.1996**
(21) Application number: 91907885.7
(22) Date of filing: 28.03.1991
(51) Int. Cl.: A61M 1/34, A61M 5/168

(54) **MULTIPLE FLUID SOURCE ISOLATION, METERING AND ALARM SYSTEM AND METHOD**
ISOLATIONS-, MESS- UND ALARMSYSTEM FÜR FLÜSSIGKEIT VON MEHREREN QUELLEN SOWIE ENTSPRECHENDES VERFAHREN
SYSTEME D'ISOLATION, DE REGULATION ET DE DOSAGE, AVEC DISPOSITIF D'ALARME, DE SOURCES MULTIPLES DE LIQUIDES ET PRINCIPE DE FONCTIONNEMENT

(30) Priority: 30.03.1990 US 502395
(43) Date of publication of application: 13.01.1993
(60) Divisional application: 95203214.2
(73) Proprietor: BAXTER INTERNATIONAL INC., Deerfield, IL 60015 (US)
(72) Inventor: FORD, Michael, G., Orange, CA 92669 (US)
(74) Representative: MacGregor, Gordon
(86) International application number: US9102192
(87) International publication number: WO9115253

(56) References cited:
- DE-C- 3 637 771
- FR-A- 2 397 197
- GB-A- 2 128 882
- Biomedizinische Technik, volume 35, no. 1/2, January/February 1990, (Berlin, DE), H. Merz et al.: "Ein steriles, geregeltes, pulsationsarmes Präzisionsdosiersystem für die Biomedizintechnik", pages 5-9, see figure 1; page 5, left-hand column, line 14 - page 6, left-hand column, line 4

## Description

### Background of the Invention

### 1. Field of the Invention

This invention relates to a fluid flow system for metering and monitoring flow from multiple liquid sources and more particularly to a plasmapheresis blood flow control system which meters and monitors liquid flow from several sources isolated from handling noise and gives advanced warning when fluid sources are depleted.

### 2. Discussion of the Prior Art

In fluid flow systems the need arises for controlling sequential or simultaneous influx from multiple fluid sources, especially those of limited capacity. In some cases it is desirable to monitor the total influx from such fluid sources so as to match the amount of fluid withdrawn from the system at another point or in another operation. For instance, plasmapheresis systems are known which require the withdrawal from and subsequent reinfusion of bodily fluids to a living subject in known or fixed amounts. The subject is usually a human or animal, but might also be a cadaver.

In the case of a plasmapheresis system, whole blood is extracted from the subject, plasma is separated from the whole blood, and an extraction product containing a higher concentration of blood cells than the whole blood is reinfused into the subject while the separated plasma is retained and used for desired purposes, treated to alleviate an undesirable condition, or discarded as unfit for further use. To compensate for plasma removed from the patient, a selected volume of replacement fluid is infused into the patient to replace the volume of plasma separated from the whole blood. The use of replacement fluids often has a therapeutic aim. Many undesirable components of blood, including diseased cells, antigens, and the like, are held in suspension therein. During therapeutic plasma exchange, these undesirable components are separated from the blood and removed with the plasma fraction, leaving the blood cleansed and healthier. The replacement fluids used during therapeutic plasma exchange can comprise a variety of fluids, such as saline, thawed plasma, and various therapeutic fluids, depending upon the desires of the treating physician. In such a case it is often desirable to control the amount and rate of each of the replacement fluids administered to the patient.

Determining the exact amount of any given replacement fluid administered to the patient during therapeutic plasma exchange is complicated by the use in modern plasmapheresis machines of disposable plastic tubing harness sets. Although manufactured to specifications as exact as possible, the flow characteristics of each plastic harness set differ somewhat from those of all others.

Therefore, despite all efforts to calibrate the peristaltic pumps with which the harness sets are used to provide sterile, non- invasive therapy, the pumps deliver at slightly different rates dependent upon the individual flow characteristics of the harness set used. The need exists, therefore, for a method of monitoring and controlling with increased precision the amount of each replacement fluid used during therapy.

Yet another problem inherent in monitoring the weight of process fluids comes from inaccuracies introduced due to motion of the fluids being monitored. For instance, the motion of replacement fluids flowing from a weighing bag is sufficient to affect the accuracy of delicate balances used in weighing by mechanical means. The need exists, therefore, for a way to isolate the fluids weighed from the effects of mass transport.

To optimize use of processing equipment and support personnel while minimizing inconvenience and discomfort to the patient, it is also desirable to reinfuse bodily fluids as rapidly and safely as possible. However, replacement fluids, usually saline, are commonly prepackaged in sterile containers of predetermined volume, necessitating that more than one container of replacement fluid be infused into the patient to replace the plasma removed during a typical session of plasmapheresis. If the containers of saline are fed sequentially into the replacement line for reinfusion, an attendant must monitor the flow of replacement fluid, and manually switch flow from one emptying bag to the next full bag before air enters the replacement line from an empty bag. In some cases the attendant must momentarily stop the flow of replacement fluid to switch flow to a new bag.

If air is inadvertently allowed to enter the infusion line from an empty bag, the machine must be stopped so that air can be removed from the flow line before it enters the patient's blood. In some cases, the entire plasmapheresis session must be scrapped and begun anew. Frequently, if a large amount of blood has been withdrawn, the patient cannot continue with a new session until sufficient time has elapsed to recuperate from the effects of the first session. Time and effort are lost while the patient is exposed to an unnecessary health risk.

The problem of replacement fluid sources going dry is compounded when multiple fluids at different rates and from separate sources are infused either sequentially or simultaneously through the single venipuncture needle. For instance, typically the reinfusion mixture contains concentrated red blood cells and sufficient anticoagulant to prevent coagulation of the red cells. At the same time, a replacement fluid, usually saline, is introduced at a rate sufficient to substitute for the plasma removed. Under certain circumstances it may be desirable to add another fluid to the mixture, such as albumin, frozen plasma, a medicament, or the like. The need exists, therefore, for a method and apparatus adapted to prevent multiple reinfusion fluid sources from going dry while monitoring the total amounts and/or relative infusion rates of the multiple reinfusion sources.

The art has long sought apparatus and equipment useful for monitoring the flow of liquid systems. It is known to monitor the flow of liquids into a fluid flow system by use of various devices. For example, in U.S. Patent 4,655,742 and European patent application No. 232,263, optical detectors are used to determine when a container of fluids is full. Automatic weighing can also be effected by means of an electrical load cell that actuates an electronic device to squeeze off a tube and thereby prevent further filling of a container as disclosed in German patent No. DE 3 739 240.

Weight scales are also known for measuring the flow of blood into or out of a container. For instance, German patent No. DE 3 737 304 discloses a weighing pan connected to a pivot such that at a certain weight of blood in a bag resting on the weighing pan, a compression valve is activated to choke the flow of blood into the bag or interrupt it intermittently. In this way, unnecessary load upon the blood donor's circulatory system and heart are avoided. In addition, European Patent No. 329786, discloses a blood separation device that holds and weighs at least two separation bags that communicate with the blood bag by means of a tube. Alternatively, as disclosed in Spanish Patent No. 8 801 535, a mechanical balancing system monitoring the difference between a total instantaneous weight and an instantaneous equilibrium force can be used to regulate the flow of substitute fluid in a blood filtration device.

"Einsatz einer mikroprozessorgeregelten Dosiereinrichtung in der Biotechnologie", Memmert *et al*, Chem Ing. Tech. 59 (1987), No. 6, pp.501-504 discloses an apparatus used for anaerobic sewage treatment and includes a first fluid container connected by a fluid flow line to a second container, with a weighing means provided for weighing the contents of the second container so that the fluid flow rate can be metered.

None of the above prior art devices warns the attendant when the replacement fluid source is going to run dry, provides a means for switching to an auxiliary source without temporarily stopping flow of replacement fluid into the reinfusion mixture, and/or meters the total amount of replacement fluid used from multiple sources. Thus, the need exists for new and better methods and apparatus for monitoring fluid flow systems, especially in plasmapheresis devices used for reinfusing replacement fluids to compensate for plasma removed and not reinfused.

The present invention relates to an apparatus for monitoring the weight of fluid flow from containers, as set out in claim 1. The precharacterising part of claim 1 is based on the Memmert et al document, and the distinguishing features of the present invention are set out in the characterising part of claim 1.

One embodiment relates to a multiple fluid flow isolation, control and alarm apparatus, which controls monitors, and meters continuous flow of fluids, usually into a fluid flow system, from a succession of containers or from multiple fluid containers. The flow control and alarm system, which is isolated from the effects of handling noise, prevents air from entering the system from an emptied container without the need for continuous visual surveillance by an attendant. The multiple fluid flow isolation, control and alarm system comprises at least one fluid container, an input fluid flow line connected at one end to the exit from the container and at the other end to a weighing bag for receiving fluids from the container(s), an output fluid flow line connected to the exit from the weighing bag, clamp means on said input fluid flow line for starting and stopping flow through said input fluid flow line, weighing means disposed for weighing the isolated fluid contents of said weighing bag; and alarm means connected to said weighing means for initiating an alarm signal when the weight of said weighing bag drops below a preselected warning level amount.

The multiple fluid flow isolation, control and alarm system can be adapted for monitoring the flow of fluids into a fluid flow system wherein it is desirable to control the exact amount of the fluids added to the flow system.

The flow control system is adapted to receive a predetermined amount of simultaneous or sequential flow from any number of containers. Preferably the containers are held by a holder so that fluid from the containers flows by gravity into the weighing bag. Replacement fluids are metered into the flow system from the weighing bag. The capacity of the weighing bag is sufficient to accommodate this function.

Preferably, to implement the warning features of the system, the weighing bag is large enough to allow continuous metering therefrom of replacement fluids after a warning alarm has been initiated and while the attendant replenishes the diminishing supply of fluid therein. Thus, the operation of the plasmapheresis machine need not be halted while the weighing bag is being replenished. In the alternative, to avoid the effects of mass transport on the weighing mechanism, in one embodiment flow from the weighing bag can be halted while its contents are replenished to the desired weight.

Fluids held in the weighing bag are metered therefrom under the operation of a control means equipped to initiate an audible or visual alarm signal when the weight of fluid in the weighing bag drops below a first higher warning level and to shut down the entire plasmapheresis operation or just the flow of replacement fluid into the system when the weight of fluid in the weighing bag drops below a second lower warning level. Successive batches of one of more fluids can be metered through the weighing bag while by these means the weighing bag is prevented from becoming empty, and air is thereby prevented from entering the flow line leading therefrom.

The cumulative amount of fluids metered through the weighing bag can be determined with great accuracy by two different methods. By the first method, flow from the weighing bag is temporarily halted when successive batches of fluids are flowed into the weighing bag and the sum total of the fluids passed through the bag is found by adding together the weights of successive batches of fluid passed into the weighing bag before flow therefrom is reinitiated.

The second method is used when continuous flow from the weighing bag is desired. By this method the cumulative amount of fluid passed through the weighing bag is determined by metering continuous flow from the weighing bag through a calibrated pump. The latter method of calculating continuous fluid flow is complicated when fluids are metered through the weighing bag by means of non-invasive peristaltic pumps and the flow lines comprise disposable plastic tubings with unique flow characteristics, as are typically used in plasmapheresis. In such cases, an absolute calibration of pump performance for the replacement fluid pump can be obtained, before or at any time during the plasmapheresis operation, using the multiple fluid flow isolation, control and alarm system herein. To calibrate the fluid replacement pump, the times required at different flow rates for a given weight of fluid of known density to pass out of the weighing bag by means of the pump and plastic set is observed. The weighing bag is allowed to fill to its maximum working weight and the clamp on the input line to the weighing bag is then closed. Thus the change in weight of fluid in the weighing bag is due solely to the operation of the pump. Using this data, the weight of replacement fluid pumped from the weighing bag at a given pump flow rate setting during any period of time can then be calculated from the time of continuous operation of the pump.

In the preferred embodiment, during normal operations the weight of fluid in the weighing bag cycles between upper and lower working level limits preselected for convenience, for example between an upper limit of 200 grams and a lower working limit of 100 grams. At the start of operations, the weighing bag is filled to the upper limit by opening the clamp on the input line and allowing an influx of fluid therein. Then the clamp is closed, the fluid pump is started, and the weight of fluid in the weighing bag continuously decreases down to the lower working limit, at which point the clamp on the input line is opened and fluid again flows into the weighing bag until the upper limit is reached, at which point the clamp again closes. In this embodiment, the pump can be automatically recalibrated each time the weight of fluid in the weighing bag moves from the upper to the lower working limit. The amount of fluid pumped during the next following filling period (while the weight of fluid moves from the lower to the upper working limit) is then calculated upon the basis of the immediately preceding pump calibration. In this way, the pump can be continually recalibrated to compensate for changes in the resiliency of the tubing set caused by temperature change, and the like.

The preferred multiple fluid flow system herein provides an automatic warning alarm when the weight of fluid in the weighing bag drops below a certain predetermined warning level selected to be below the lower working limit. The attendant is given sufficient warning before the weighing bag empties that the supply of replacement fluids in the weighing bag can be replenished, manually if necessary, without shutting down the flow of fluids through the system. If the weight of the weighing bag continues to diminish, for instance, because the supply of fluid therein has not been replenished, at a second, lower predetermined fail safe weight of fluid the automatic fail-safe feature of the system shuts down all operations or stops the flow of the replacement fluids.

The preferred flow control and alarm system herein also provides an automatic self-checking feature to eliminate the possible hazards inherent in automatically controlled operations. This feature automatically generates an error signal when, according to the most recent pump calibration, the pump has been running long enough to reduce the weight of the weighing bag from the upper working level to below the lower working level but the actual weight of fluid in the weighing bag has not decreased accordingly.

In the preferred embodiment, the multiple fluid flow isolation, control and alarm system is adapted to continuously meter known, exact amounts of replacement fluid, into a disposable plastic flow set of the type used with a typical plasmapheresis machine. The replacement fluid, usually several aliquots of saline from pre-packaged, sterile containers, usually holding about 500 to 1000 milliliters of fluid each, is added without allowing air to enter the flow set and mix with the blood to be returned to the patient. Other fluids can also be added either mixed with the saline or separately.

In addition to the multiple fluid flow isolation, control and alarm system described in detail herein, the flow set, sometimes known as a "harness set", includes a blood separator to separate out and collect a blood component while returning to the donor or patient the balance of the blood, along with sufficient replacement fluid to compensate for the component removed. When blood withdrawal and fluid reinfusion is cyclic, a single attachment means to the patient is used, such as a single phlebotomy needle. On the other hand, when both operations proceed simultaneously, the flow set includes two means of attachment to the donor or patient, such as separate withdrawal and reinfusion phlebotomy needles.

### Brief Description of the Drawings

A better understanding of the invention can be had from a consideration of the following detailed description, taken in conjunction with the accompanying drawings in which:
Fig. 1 is a schematic and block diagram representation of a fluid flow path for a plasmapheresis system using a multiple fluid source isolation, metering, and alarm system in accordance with the invention;

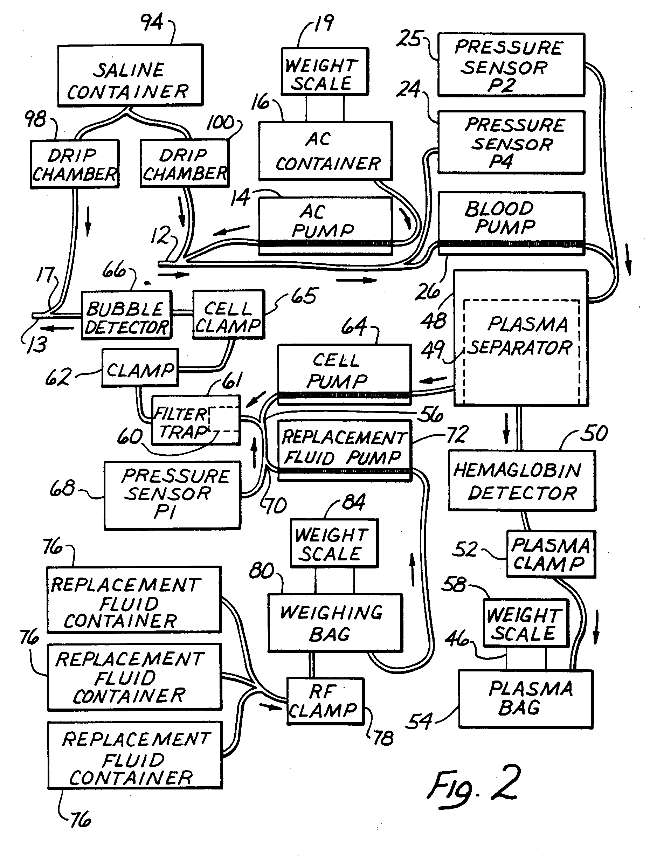

Fig. 2 is a functional block diagram representation of a plasmapheresis system incorporating a multiple fluid source isolation, metering, and alarm system in accordance with the invention;
Fig. 3 is a schematic and block diagram representation of a fluid flow path for a plasmapheresis system using a multiple fluid source isolation, metering, and alarm system in accordance with the invention wherein multiple fluid sources are connected to the weighing bag.

### Detailed Description of the Preferred Embodiment

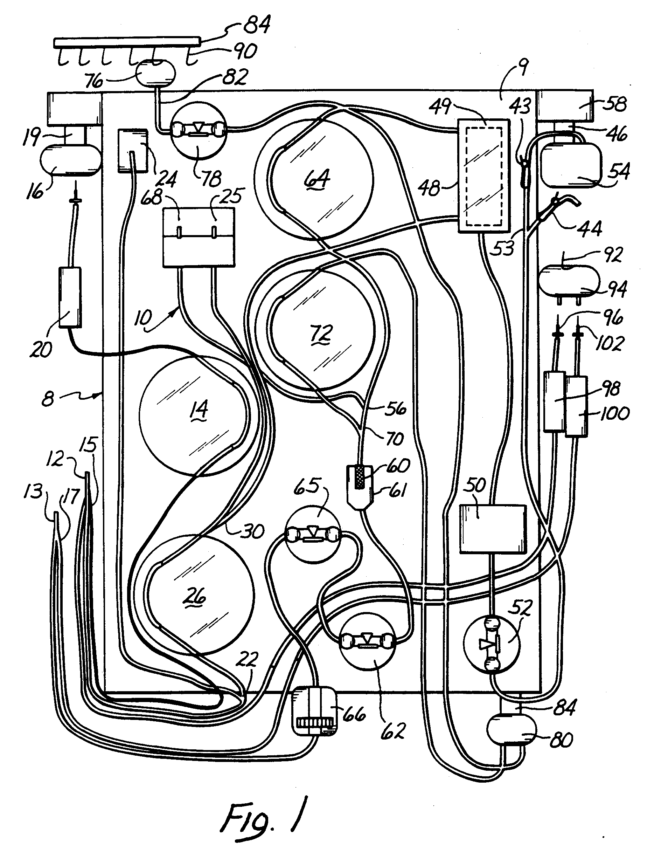

Referring now to Fig. 1, a conventional plasmapheresis instrument is generally designated 8 and includes various pumps, clamps, detectors, monitoring systems, indicators, and the like, not all of which are described in the present application or necessary to an understanding of the present invention. Therefore, only those parts of the instrument which are applicable for an understanding of the present invention will be described.

Instrument 8, on its face 9, includes four peristaltic pumps which are individually driven and under the control of a computerized control system. A disposable harness set is applied to the instrument and to the donor such that blood collection, separation and infusion of packed cells with replacement fluid can be provided. Flow path 10 of the disposable harness set will now be described.

Flow path 10 provides a noninvasive, sterile plasmapheresis flow path for a dual needle plasmapheresis system utilizing a multiple fluid flow isolation, control and alarm system in accordance with the invention. Intravenous connection of flow path 10 to a subject is provided by dual bodily fluid flow channel connections such as phlebotomy needles 12 and 13 which are suitable for insertion into the veins of a living (or deceased) subject to provide bidirectional communication of blood and other fluids between the subject and flow path 10 of the plasmapheresis system.

The flow path branches immediately adjacent withdrawal needle 12 at branch point 15 with one branch extending through a noninvasive peristaltic anticoagulant pump 14 and drip chamber 20 to an anticoagulant container 16 held by holder 19. During operation, anticoagulant pump 14 operates to supply and mix a small percentage of anticoagulant with the blood as it is being extracted to prevent activation of clotting mechanisms that would cause the blood to cling to tubing sidewalls as it passes through flow path 10. By mixing the anticoagulant with the whole blood at needle 12, the two fluids become fully mixed during withdrawal and less anticoagulant is required. This desirable result helps minimize the amount of anticoagulant introduced into the blood.

The other branch of blood flow path 10 leaving withdrawal needle 12 extends to another branch point 22. Optionally, from branch point 22 one branch extends to a pressure sensor 24 coupled to sense fluid pressure on the subject side of a blood pump 26. The pressure sensor 24 includes a disposable filter coupling the sensor to a pressure sensor tube (not shown) so as to maintain a noninvaded sterile atmosphere within the flow path 10. The second branch from branch point 22 extends through the noninvasive, peristaltic blood pump 26 to branch point 30. From branch point 30 one branch leads to pressure sensor 25, which also includes a disposable filter coupling to maintain sterility. Pressure sensor 25 detects pressure across filter 49 in separator 48. Another flow path from branch point 30 extends to the bottom of plasma separator 48, which encloses filter 49.

While the exact nature of the plasma separator 48 is not material to the present invention and can be fully conventional if desired, a highly advantageous plasma separator is a centrifugal filter type of separator as illustrated in U.S. Patent No. 5194145 No. For this type of separator the end product plasma output is coupled through a hemoglobin detector 50 and a plasma clamp 52 through branch point 53 and clamp means 43, such as a roller clamp, to a plasma container 54, which is maintained at atmospheric pressure. The plasma container 54 is suspended from a hanger means 46 of a weight scale 58, which provides feedback to the plasmapheresis system of the amount of plasma within container 54. Another open ended flow path from branch point 53 extends through clamp means 44, such as a roller clamp, for attaching an auxiliary plasma container (not shown) to flow path 10.

A cell pump 64 coupled with an outlet of plasma separator 48 controls the reinfusion flow of high hematocrit blood from plasma separator 48 through a flow path extending through branch points 56, and 70, filter 60 and flexible bubble trap 61, blood clamps 62 and 65, air detector 66, branch point 17, and reinfusion needle 13. From branch point 56 another flow path extends to pressure sensor 68. Since plasma removed from separator 48 is maintained at atmospheric pressure plus a small adjustment for vertical height differences, the difference between pressure sensors 25 and plasma container 54 provides an indication of pressure across filter 49 within plasma separator 48. This pressure indication can be useful in monitoring and controlling the operation of plasma separator 48.

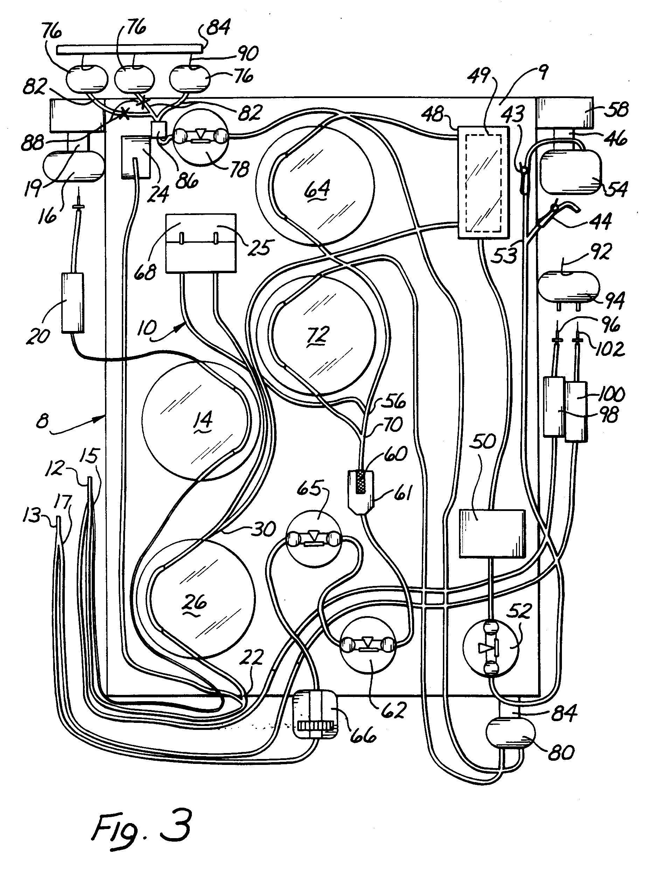

From branch point 70, another flow path extends through replacement fluid pump 72 to replacement fluid weighing bag 80. Weighing bag 80 is suspended from scale means 84, and another flow path extends from weighing bag 80 through clamp 78 and connector 82 to one of a plurality of fluid containers 76. In an alternative embodiment, as shown in Fig. 3, from clamp 78 the flow path extends to drip chamber 86 at the exit from which it branches into a plurality of flow paths extending to a plurality of connectors 82, such as a plastic spikes, and individual fluid containers 76. A clamp means 88 is located along each reinfusion line 82 for independently controlling the flow of fluid therethrough to weighing bag 80. In drip chamber 86 fluids from any or all of containers 76 are received and mixed. Usually at least one of fluid containers 76 is filled with saline. Others of the fluid containers 76 can also be filled with supplementary bags of saline. In both embodiments shown in Figs. 1 and 3, fluid containers 76 are suspended or supportably attached by attachment means 90 to replacement fluid holder 84.

Returning now to Figure 1, from branch point 15 a third branch of the flow line extends through drip chamber 98 and connector 96 to saline bag 94 suspended from attachment means 92. From branch point 17 near reinfusion needle 13 a second branch of the flow path extends through drip chamber 100 and connector 102 to saline bag 94. Before and after the plasmapheresis cycle, saline from fluid container 94 can be passed through needles 12 and/or 13 to prime or cleanse the needle and thereby prevent clogging due to coagulation of blood therein. This flow path enables the separator to be primed with a small amount of saline prior to initial use and to be cleansed with saline after final use. If for any reason the plasmapheresis cycle is temporarily halted, saline can be dripped through needles 12 and 13 to keep them open until operations are reinitiated. The saline drip preferably can be initiated manually by the attendant opening control means (not shown), such as a roller clamp. Alternatively, the computerized control means can initiate the saline drip therethrough whenever the fail safe mechanism shuts down operations, as further described hereinbelow.

During normal operations the weight of fluid in the weighing bag cycles between upper and lower working level limits preselected for convenience, for example between an upper limit of 200 grams and a lower working limit of 100 grams. To initiate the cycle at the start of operations, after the system has been primed and the pump 72 has been initially calibrated, the weighing bag 80 is filled to the upper limit by opening the clamp 78 on the input line and allowing an influx of fluid therein. Then the clamp 78 is closed, pump 72 is started, and the weight of fluid in the weighing bag continuously decreases down to the lower working limit, at which point the clamp on the input line is opened and fluid again flows into the weighing bag until the upper limit is reached, at which point the clamp again closes. In this embodiment of the invention, the pump can be automatically recalibrated each time the weight of fluid in the weighing bag moves from the upper to the lower working limit. The amount of fluid pumped during the next following filling period (while the weight of fluid moves from the lower to the upper working limit) is then calculated upon the basis of the immediately preceding pump calibration. In this way, the pump can be continually recalibrated to compensate for changes in the resiliency of the tubing set caused by temperature change, and the like.

During plasmapheresis, using the apparatus and method of this invention, blood from the patient is withdrawn through needle 12, and sent through separator 48 for removal of plasma therefrom. Replacement fluids are simultaneously withdrawn from weighing bag 80 via pump 72, mixed with red cell concentrate at branch point 70 (pumped via pump 64 from the separator 48 outlet), and reinfused to the patient via needle 13. When used for plasma replacement therapy, plasma usually is considered waste and discarded; however, the plasma can also be retained and given therapeutic treatment by known means. Although one skilled in the art will appreciate that the apparatus could be modified to perform cyclic withdrawal and reinfusion functions, preferably withdrawal, separation and reinfusion proceed simultaneously. In either the cyclic or sequential modes of operation, the reinfusion mixture returned to the patient via reinfusion needle 13 comprises concentrated cells of increased hematocrit and anticoagulant recovered from the separator 48 and sufficient replacement fluid from individual replacement bag(s) 76 to replace in any proportion the amount of plasma collected in weighed plasma bag 54 as directed by the treating physician.

Attached to scale means 84 is an alarm means, not shown, for providing a visual or audible signal whenever during plasmapheresis the amount of fluid contained in weighing bag 80 drops below a preselected relatively low warning amount, preferably from about 75 to 85 grams. In addition, scale means 84 is provided with an automatic fail safe means (not shown) for shutting down the plasmapheresis operation whenever the amount of fluid in weighing bag 80 drops below a second, lower preselected fail safe amount. The difference between the warning amount and the fail safe amount is any convenient amount selected to allow the attendant sufficient time to add additional fluids to weighing bag 80 while pump 72 continues to run before it empties to the fail safe amount. The exact amount of the difference between the warning and fail safe amounts, of course, will usually differ depending upon the rate of pump 72.

The control and alarm system herein also provides an automatic self-checking feature to eliminate the possible hazards inherent in automatically controlled operations. The control means automatically generates an audible or visual error signal when pump 72 has been running with clamp means 78 closed long enough to deplete the weight of fluid in the weighing bag some preselected amount, preferably from the maximum working weight at initiation of operations, but the actual weight of fluid in the weighing bag has not dropped a corresponding amount.

In use, the harness set comprising flow path 10 is applied to face 9 of instrument 8 as illustrated in Figure 1. Separator 48 is placed into a motor mount (not shown) and flow path 10 is threaded into the anticoagulant pump 14, blood pump 26, cell pump 64, and replacement fluid pump 72, as well as clamps 78, 65, 62, and 52. Weighing bag 80 is hung from scale 84 and plasma bag 46 is hung from scale 58. Replacement fluid bags 76 are hung from attachment means 90 and at least one of them is attached to flow path 10 using a connector 82. Saline bag 94 is hung from attachment means 92 and attached to flow path 10 via connectors 96 and 102.

In operation, various set-up and safety procedures are followed and the attachment means 12 and 13 are phlebotomy needles applied to the patient. To utilize the multiple fluid flow isolation, control and alarm system features of the invention, clamp 78 is opened and a known quantity of replacement fluid from one or more of the replacement fluid bags 76 is allowed to circulate, for example by gravity flow, into replacement fluid weighing bag 80. Clamp 78 is then closed. The amount of fluid circulated into weighing bag 80 is sufficient to allow plasmapheresis operations to proceed at least until an additional isolated aliquot or batch of replacement fluids can be circulated into weighing bag 80 by repeating the above steps, usually at least 100 grams or more. Preferably, however, no more than between about 100 and 200 grams at a time are circulated into weighing bag 80 to facilitate switching from one replacement fluid type to another without emptying weighing bag 80. Optionally several of bags 76 can contain the same type of fluid and connector 82 can be moved from one to the next to empty several of bags 76 into weighing bag 80 in a single batch.

Plasmapheresis is initiated by opening clamps 52, 62 and 65 and starting pumps 26, 14, 64 and 72. Blood from the patient mixed with anticoagulant in proportions controlled by the relative rates of pumps 14 and 26 flows to separator 48 wherein plasma is separated and circulated to plasma bag 54. Simultaneously, concentrated cells recovered from separator 48 are circulated by pump 64 to branch point 70 where they are joined by a flow of replacement fluid from weighing bag 80 at a rate controlled by pump 72. The replacement fluid is usually delivered in some predetermined ratio to the amount of plasma being removed to plasma bag 54. When the amount of fluid in weighing bag 80 drops below the preselected warning amount, the alarm attached to weighing bag 80 is triggered to produce either an audible or visible alarm.

The alarm means and fail safe means attached to weighing means 84 can utilize known methods of triggering mechanical alarm. Preferably, however, the alarm and fail safe means are incorporated within a programmed digital processor that controls the operation of the plasmapheresis machine using known principles.

The warning alarm alerts the attendant that replacement fluid weighing bag 80 is approaching empty. In response to the warning alarm, the attendant can replenish the replacement fluid in bag 80 before it runs dry by either of two methods. By the first, less preferred method the attendant stops either pump 72 or all of pumps 26, 14, 64 and 72, checks to see that one of the replacement fluid bag(s) 76 contains at least an aliquot of fluid, and opens the clamp 88 on the bag 76 to allow fluids therefrom to empty into weighing bag 80 until the weight of replacement fluid in weighing bag 80 is increased above the warning level and the pump or pumps are all restarted. By the second, more preferred method the pumps are not stopped and the contents of bag 80 continue to empty while the auxiliary fluid replacement bags 76 are positioned by the attendant and emptied as described above into bag 80.

If the amount of replacement fluid in weighing bag 80 is not replenished before the weight of fluid therein drops to the fail safe amount, the automatic fail safe means associated with weighing means 84 shuts down operations by stopping either pump 72 alone or pumps 14, 26, 64, and 72 and closing clamps 52, 62 and 64, and generates a fail safe alarm message for the attendant. Once the control means has shut down operations, the control means stops or removes the fail safe alarm and operations of the system can be reinitiated by starting the pump or pumps and opening clamps 72, 62, and 64 when the replacement fluids in weighing bag 80 have been replenished to some predetermined reinitiation amount selected to be intermediate between the warning amount and the fail safe amount, preferably at least 30 grams above the fail safe amount. When the weight of fluid in the weighing bag rises above the reinitiation amount, system operations are reinitiated either manually in response to cessation or removal of the fail safe alarm, or automatically by the control means.

This fail safe feature prevents weighing bag 80 from completely emptying so that air never enters flow path 10 from bags 76 or from weighing bag 80. Therefore, once the amount of fluid in bag 80 has been replenished, the system operation can be continued and does not have to be scrapped.

The flow characteristics of each plastic set are necessarily unique due to slight variations in internal diameter, and the cumulative effect of connectors and branch points upon flow dynamics. Thus the performance of pump 72 is different with each flow set used and can change during the course of the procedure. Despite these differences, replacement fluid pump 72 can readily be calibrated to take into account the individual flow characteristics of flow path 10 using the multiple fluid flow isolation, control and alarm system herein. To accomplish this, either before or during plasmapheresis, with clamp means 78 closed, the time required to pump any known amount of fluid from weighing bag 80 through the associated portions of flow path 10 is noted and the rate of delivery of pump 72 is readily derived by known means.

Absolute calibration of pump 72 by this method provides the advantage that the total amount of replacement fluid delivered to the patient from the replacement fluid bags 76 can be determined with great accuracy whether the replacement fluid consists essentially of a single fluid, such as saline, or whether it comprises a mixture of saline and other therapeutic fluids, such as liquid antibiotics and the like. Thus, the physician's instructions regarding the needs of the patient can be accomplished with great accuracy despite the effect of the individual flow characteristics of the harness set upon the performance of pump 72. Preferably the system is recalibrated during each weighing bag cycle during the time the weight in weighing bag 80 moves from the upper working limit to the lower working limit and the pump calibration thus obtained is used to compute the amount of fluid delivered by pump 72 during the next following fill cycle, while the weight in the weighing bag 80 moves between the lower working limit and the upper working limit.

## Claims

1. An apparatus for monitoring the weight of fluid flow from containers, said apparatus comprising: at least one replacement fluid container (76) of limited capacity; an input fluid flow line (82) connected at one end to the exit from the container and to a weighing bag (80) at the other end; an output fluid flow line having an inlet and an outlet, the inlet being connected to the exit from the weighing bag (80); and weighing means (84) disposed for weighing the fluid contents of said weighing bag (80); characterised by alarm means connected to said weighing means (84) for initiating an alarm signal when the weight of said weighing bag (80) drops below a preselected warning amount; means (82) to preconnect a plurality of replacement fluid containers (76) to said input fluid flow line (82); and clamp means (78) on said input fluid flow line for starting and stopping flow through said fluid flow line.

2. The apparatus of claim 1 further comprising an automatic control means connected to said weighing means (84) for controlling flow into and out of the weighing bag (80).

3. The apparatus of claim 1, wherein the control means includes fail safe means connected to said weighing means for shutting down flow from said weighing bag (80) when the weight of fluid in the bag drops below a preselected lower working limit amount, the warning amount being higher than the lower working limit amount.

4. The apparatus of claim 3, wherein the fail safe means is further connected to the clamp (78) and automatically opens the clamp when the weight of fluid in the weighing bag (80) reaches the lower working limit amount and automatically closes the clamp (78) when the weight of fluid in the weighing bag reaches an upper working limit amount.

5. The apparatus of claim 3 or 4, wherein the control means is adapted to initiate the warning alarm when weight of fluid in the weighing bag drops below a second warning amount, the second warning amount being below the lower working limit amount.

6. The apparatus of claim 3, 4 or 5 wherein the lower working limit amount is 15 grams of fluid.

7. The apparatus of any one of claims 3 to 6 wherein the control means is adapted to reinitiate flow from the weighing bag (80) after a fail safe shut down, when the fluid in the weighing bag (80) rises above a preselected reinitiation amount between the warning amount and the lower working limit amount.

8. The apparatus of claim 7 wherein the preselected reinitiation amount is at least 30 grams above the lower working limit amount.

9. The apparatus of any preceding claim wherein the or the first warning amount is from 75 to 85 grams of fluid.

10. The apparatus of any preceding claim including a pump (72) in the output fluid flow line, the control means being adapted to control the pump (72) for removing fluid from the weighing bag (80) and generate an error signal when the pump (72) has operated for a period of time and at a rate sufficient to remove a preselected amount of fluid from the weighing bag (80), but the weight of fluid in the weighing bag (80) has not dropped a corresponding amount.

11. The apparatus of claim 10 wherein the control means is adapted to generate an error signal when the pump (72) has operated at a rate sufficient to remove 150 grams of fluid from the weighing bag (80).

12. The apparatus of any preceding claim wherein a plurality of fluid containers (76) is connected to the input fluid flow line (82).

13. The apparatus of any preceding claim wherein a drip chamber (86) is located on said input fluid flow line (82) for removing bubbles from fluids introduced into the fluid flow system.

14. The apparatus of any preceding claim wherein said apparatus further comprises fluid holder means (90) adapted to hold or holding a plurality of fluid containers (76) of limited capacity so that at least one of said containers is connected to said weighing bag (80), via said input flow line (82)

15. The apparatus of claim 14 wherein fluid from the containers (76) flows by gravity into the weighing bag (80).

16. The apparatus of claim 14 or 15 wherein each replacement fluid container (76) is attached to the entrance into a drip trap (86) and the exit from the drip trap is attached to the input fluid flow line (82).

17. The apparatus of any one of claims 3 to 8 wherein the control means is operable to monitor the total weight of replacement fluid passed from the supply source to the output fluid flow line.

18. An assembly for monitoring the amount of fluid flow from replacement fluid containers into a disposable flow line for use in plasmapheresis, said assembly comprising an apparatus as in any preceding claim with the outlet of the output fluid flow line being connected to a disposable plasmapheresis fluid flow apparatus for withdrawing whole blood from a subject, separating out and collecting a first blood fraction therefrom and for reinfusing into the subject a mixture of replacement fluid and the remaining blood fraction.

19. An assembly comprising an apparatus as in any one of claims 1 to 17, with the outlet of the output fluid flow line being connected to a fluid flow apparatus.

20. An assembly as in claim 18 or 19, for use as a plasma exchange system for separating blood received from a subject into constituents and infusing the subject with a first blood constituent and a replacement fluid, said fluid flow apparatus comprising: a separator (48) for separating first and second blood constituents from whole blood; an attachment means (12) for continuously withdrawing whole blood from a subject and supplying the blood to the separator (48); means (16,20,14,15) for introducing anticoagulant to the whole blood withdrawn from the subject; an attachment means (13) for continuously reinfusing the subject with the first blood constituent and a replacement fluid; flow means (12,15,22,26,30) including the withdrawal attachment means (12) for flowing the whole blood and the anticoagulant to the separator (48) and means (13,64,70,72) including the reinfusion attachment means (13) for receiving the first blood constituent from the separator (48) and the replacement fluid from the replacement fluid source (76) and for flowing the first blood constituent and the replacement fluid to the subject; the alarm means being operable to initiate a warning alarm when the supply source (76) approaches empty; and the other of said apparatuses including means (78) for sequentially delivering isolated amounts of replacement fluid to the weighing bag for sequential weighing by weighing means (84).

21. A method of operating the assembly of claim 19 so as to prevent air from entering the flow system when the, or at least one of the, replacement fluid containers (76) is empty, said method comprising:
(1) flowing a first isolated amount of fluid from the, or at least one of the, containers (76) into the weighing bag (80);
(2) weighing the fluid in the weighing bag (80);
(3) controlling the flow of fluid from the container or containers (76), such that fluid is flowed into the weighing bag (80) when the weight of fluid in the bag has dropped below a preselected lower working limit amount and fluid flow into the weighing bag is stopped when the weight of fluid in the weighing bag rises above a preselected upper working limit; and
(4) flowing fluid from the weighing bag into the fluid flow apparatus until the weight of fluid in the bag falls below the lower working limit.

22. The method of claim 21 further including the step of automatically controlling the flow of fluid from the container or containers (76) to the weighing bag (80) so that the weighing bag is replenished by fluid from the container or containers (76) without stopping operation of the fluid flow apparatus.

23. The method of claim 21 or 22 further including the steps of repeating steps (1), (2), (3) and (4) a plurality of times.

24. The method of claim 21 or 22 wherein the isolated amount of fluid comprises from 100 to 800 grams.

25. The method of claim 23 wherein each isolated amount of fluid comprises from 100 to 200 grams.

26. The method of any one of claims 21 to 25 further including the following steps:
(5) triggering the alarm means to initiate a warning signal when the weight of fluid in the weighing bag (80) drops below a second warning amount which is below the lower working limit amount; and
(6) flowing, in response to the warning signal into the weighing bag (80) of an additional isolated amount of fluid from the, or at least one of the containers so that the weight of fluid in the weighing bag (80) is raised above the second warning amount.

27. A method of operating the assembly of claim 18, so as to prevent air from entering the fluid flow apparatus when the, or at least one of the, fluid containers (76) is empty, said method comprising:
(1) flowing a first isolated amount of fluid from the, or at least one of the fluid containers (76) into the weighing bag (80);
(2) weighing the weighing bag (80);
(3) isolating the weighing bag from flow disturbances during weighing;
(4) continuously flowing fluid from the weighing bag into the fluid flow apparatus while maintaining the amount of fluid in the weighing bag (80), between upper and lower working limit amounts by replenishing the weighing bag from the, or at least one of the, containers (76).

28. The method of claim 27 wherein due to insufficient fluid source the amount of fluid in the weighing bag (80) drops below the lower working limit amount further including the steps of:
(5) generating a warning signal;
(6) flowing an additional isolated amount of fluid from at least one additional replacement fluid source into the weighing bag in response to the second alarm signal so that the weight of fluid in the weighing bag (80) remains above the lower working limit amount.

29. The method of claim 28 further including the step of ensuring that the flow out of the weighing bag (80) continues uninterrupted while step (5) is performed.

30. The method of claim 28 or 29 further including the step of temporarily interrupting flow out of the weighing bag (80) while step (5) is performed.

31. The method of any one of claims 21 to 30 further including the steps of:
triggering the alarm means to initiate a warning signal when the weight of fluid in the weighing bag drops below a second warning amount, the second warning amount being below the lower working limit amount;
shutting down flow from the weighing bag (80) in response to the warning signal;
flowing sufficient of an additional isolated amount of fluid from at least one additional replacement fluid source (76) into the weighing bag (80) to raise the weight of fluid therein above the first warning amount; and
reinitiating flow from said weighing bag (80) into said fluid flow apparatus.

32. The method of claim 31 further including the step of automatically reinitiating flow from the weighing bag (80) into the fluid flow apparatus when the weight of fluid in the weighing bag rises to a preselected reinitiation amount after the shut down step.

33. The method of claim 31 or 32 wherein each isolated amount of replacement fluid comprises from 100 to 200 grams of fluid.

34. The method of claim 33 wherein the first higher warning amount is from 65 to 85 grams.

35. The method of claim 32 wherein the second warning amount is below 15 grams, and including the step of automatically reinitiating fluid flow when the weight of fluid in the weighing bag rises above the reinitiation amount.

36. The method of any one of claims 26 to 35 further including the step of adding together the weights of the isolated amounts of fluid flowed into the weighing bag to compute the total amount of fluid flowed therefrom into the flow system.

## Patentansprüche

1. Vorrichtung zur Überwachung des Gewichtes von Fluidströmungen aus Behältern, wobei die Vorrichtung folgendes aufweist:
- mindestens einen Ersatzfluidbehälter (76) mit begrenzter Kapazität;
- eine Einlaß-Fluidströmungsleitung (82), die an dem einen Ende an den Ausgang des Behälters und an dem anderen Ende an einen Wägebeutel (80) angeschlossen ist;
- eine Auslaß-Fluidströmungsleitung mit einem Einlaß und mit einem Auslaß, wobei der Einlaß mit dem Ausgang des Wägebeutels (80) verbunden ist; und
- eine Wägeeinrichtung (84), die zum Wägen des Fluidinhalts des Wägebeutels (80) vorgesehen ist,
gekennzeichnet durch
eine Alarmeinrichtung, die an die Wägeeinrichtung (84) angeschlossen ist, um ein Alarmsignal auszulösen, wenn das Gewicht des Wägebeutels (80) unter einen vorgewählten Alarmwert absinkt;
eine Einrichtung (82), um eine vielzahl von Ersatzfluidbehältern (76) vorher mit der Einlaß-Fluidströmungsleitung (82) zu verbinden; und
eine Klemmeinrichtung (78) an der Einlaß-Fluidströmungsleitung, um die Strömung durch die Fluidströmungsleitung zu starten und zu stoppen.

2. Vorrichtung nach Anspruch 1,
die ferner eine automatische Steuereinrichtung aufweist, welche an die Wägeeinrichtung (84) angeschlossen ist, um das Einströmen in den und das Ausströmen aus dem Wägebeutel (80) zu steuern.

3. Vorrichtung nach Anspruch 1,
wobei die Steuereinrichtung eine ausfallsichere Einrichtung aufweist, die an die Wägeeinrichtung angeschlossen ist, um das Ausströmen aus dem Wägebeutel (80) zu sperren, wenn das Gewicht des Fluids in dem Beutel unter einen vorgewählten unteren Arbeitsgrenzwert absinkt, wobei der Alarmwert höher ist als der untere Arbeitsgrenzwert.

4. Vorrichtung nach Anspruch 3,
wobei die ausfallsichere Einrichtung ferner an die Klemmeinrichtung (78) angeschlossen ist und die Klemmeinrichtung automatisch öffnet, wenn das Gewicht des Fluids in dem Wägebeutel (80) den unteren Arbeitsgrenzwert erreicht, und die Klemmeinrichtung (78) automatisch schließt, wenn das Gewicht des Fluids in dem Wägebeutel einen oberen Arbeitsgrenzwert erreicht.

5. Vorrichtung nach Anspruch 3 oder 4,
wobei die Steuereinrichtung so ausgelegt ist, daß sie den Alarm auslöst, wenn das Gewicht des Fluids in dem Wägebeutel unter einen zweiten Alarmwert absinkt, wobei der zweite Alarmwert niedriger ist als der untere Arbeitsgrenzwert.

6. Vorrichtung nach Anspruch 3, 4 oder 5,
wobei der untere Arbeitsgrenzwert 15 Gramm des Fluids beträgt.

7. Vorrichtung nach einem der Ansprüche 3 bis 6,
wobei die Steuereinrichtung so ausgelegt ist, daß sie nach einer Fail-Safe-Absperrung die Strömung aus dem Wägebeutel (80) wieder einleitet, wenn das Fluid in dem Wägebeutel (80) über einen vorgewählten Wiedereinleitungswert zwischen dem Alarmwert und dem unteren Arbeitsgrenzwert angestiegen ist.

8. Vorrichtung nach Anspruch 7,
wobei der vorgewählte Wiedereinleitungswert mindestens 30 Gramm über dem unteren Arbeitsgrenzwert liegt.

9. Vorrichtung nach einem der vorhergehenden Ansprüche,
wobei der oder der erste Alarmwert 75 Gramm bis 85 Gramm des Fluids beträgt.

10. Vorrichtung nach einem der vorhergehenden Ansprüche,
die eine Pumpe (72) in der Auslaß-Fluidströmungsleitung aufweist, wobei die Steuereinrichtung so ausgelegt ist, daß sie die Pumpe (72) steuert, um Fluid aus dem Wägebeutel (80) zu entfernen, und ein Fehlersignal erzeugt, wenn die Pumpe (72) für eine Zeitspanne und mit einem Durchsatz gearbeitet hat, die ausreichend sind, um eine vorgewählte Menge des Fluids aus dem Wägebeutel (80) zu entfernen, aber das Gewicht des Fluids in dem Wägebeutel (80) nicht um einen entsprechenden Betrag abgenommen hat.

11. Vorrichtung nach Anspruch 10,
wobei die Steuereinrichtung so ausgelegt ist, daß sie ein Fehlersignal erzeugt, wenn die Pumpe (72) mit einem Durchsatz gearbeitet hat, der ausreichend ist, um 150 Gramm des Fluids aus dem Wägebeutel (80) zu entfernen.

12. Vorrichtung nach einem der vorhergehenden Ansprüche,
wobei eine Vielzahl von Fluidbehältern (76) an die Einlaß-Fluidströmungsleitung (82) angeschlossen ist.

13. Vorrichtung nach einem der vorhergehenden Ansprüche,
wobei eine Tropfkammer (86) an der Einlaß-Fluidströmungsleitung (82) angebracht ist, um Blasen aus den Fluiden zu entfernen, die in das Fluidströmungssystem eingeleitet werden.

14. Vorrichtung nach einem der vorhergehenden Ansprüche,
wobei die Vorrichtung ferner eine Fluidhalteeinrichtung (90) aufweist, die eine Vielzahl von Fluidbehältern (76) mit begrenzter Kapazität halten kann oder hält, so daß zumindest einer von den Behältern über die Einlaß-Fluidströmungsleitung (82) an den Wägebeutel (80) angeschlossen ist.

15. Vorrichtung nach Anspruch 14,
wobei Fluid aus den Behältern (76) durch die Einwirkung der Schwerkraft in den Wägebeutel (80) fließt.

16. Vorrichtung nach Anspruch 14 oder 15,
wobei jeder Ersatzfluidbehälter (76) an dem Eingang in eine Tropfenfalle (86) angebracht ist und der Ausgang aus der Tropfenfalle an der Einlaß-Fluidströmungsleitung (82) angebracht ist.

17. Vorrichtung nach einem der Ansprüche 3 bis 8,
wobei die Steuereinrichtung derart betreibbar ist, daß sie das Gesamtgewicht des Ersatzfluides überwacht, welches von der Versorgungsquelle zu der Auslaß-Fluidströmungsleitung geflossen ist.

18. Anordnung zur Überwachung der Menge einer Fluidströmung aus Ersatzfluidbehältern in eine Einweg-Strömungsleitung zur Verwendung bei der Plasmapherese, wobei die Anordnung eine Vorrichtung nach einem der vorhergehenden Ansprüche aufweist, wobei der Auslaß der Auslaß-Fluidströmungsleitung an eine Einweg-Plasmapherese-Fluidströmungsvorrichtung angeschlossen ist, um Vollblut von einem Patienten zu entnehmen, daraus einen ersten Blutanteil abzutrennen und zu sammeln und dem Patienten eine Mischung aus Ersatzfluid und dem übrigen Blutanteil wieder zuzuführen.

19. Anordnung, die eine Vorrichtung nach einem der Ansprüche 1 bis 17 aufweist, wobei der Auslaß der Auslaß-Fluidströmungsleitung an eine Fluidströmungsvorrichtung angeschlossen ist.

20. Anordnung nach Anspruch 18 oder 19, zur Verwendung bei einem Plasmaaustauschsystem, um von einem Patienten erhaltenes Blut in seine Bestandteile zu trennen und dem Patienten einen ersten Blutbestandteil und ein Ersatzfluid wieder zuzuführen, wobei die Fluidströmungsvorrichtung folgendes aufweist:
- einen Separator (48), um aus dem Vollblut erste und zweite Blutbestandteile abzutrennen;
- eine Befestigungseinrichtung (12), um von einem Patienten kontinuierlich Vollblut abzuziehen und das Blut dem Separator (48) zuzuführen;
- eine Einrichtung (16, 20, 14, 15), um in das von dem Patienten abgezogene Vollblut ein Antikoagulans einzuleiten;
- eine Befestigungseinrichtung (13), um dem Patienten kontinuierlich den ersten Blutbestandteil und ein Ersatzfluid wieder zuzuführen;
- eine Strömungseinrichtung (12, 15, 22, 26, 30), welche die Entnahme-Befestigungseinrichtung (12) enthält, um das Vollblut und das Antikoagulans zu dem Separator (48) strömen zu lassen; und
- eine Einrichtung (13, 64, 70, 72), welche die Wiederzuführungs-Befestigungseinrichtung (13) enthält, um den ersten Blutbestandteil von dem Separator (48) und das Ersatzfluid von der Ersatzfluidquelle (76) zu erhalten und um den ersten Blutbestandteil und das Ersatzfluid zu dem Patienten strömen zu lassen;
wobei die Alarmeinrichtung derart betreibbar ist, daß sie ein Alarmsignal auslöst, wenn sich die Versorgungsquelle (76) dem leeren Zustand nähert; und
wobei die andere der Vorrichtungen eine Einrichtung (78) aufweist, um nacheinander getrennte Mengen des Ersatzfluids dem Wägebeutel zuzuführen, um diese mit der Wägeeinrichtung (84) sequentiell zu wägen.

21. Verfahren zum Betreiben der Anordnung nach Anspruch 19, um zu verhindern, daß Luft in das Strömungssystem eintritt, wenn die oder zumindest der eine der Ersatzfluidbehälter (76) leer sind, wobei das Verfahren folgende Schritte aufweist:
(1) man läßt eine erste getrennte Menge des Fluids aus den oder zumindest aus einem von den Behältern (76) in den Wägebeutel (80) strömen;
(2) das Fluid in dem Wägebeutel (80) wird gewogen;
(3) die Strömung des Fluids aus dem Behälter oder den Behältern (76) wird derart gesteuert, daß man Fluid in den Wägebeutel (80) strömen läßt, wenn das Gewicht des Fluids in dem Beutel unter einen vorgewählten unteren Arbeitsgrenzwert abgenommen hat, wobei die Fluidströmung in den Wägebeutel dann gestoppt wird, wenn das Gewicht des Fluids in dem Wägebeutel über einen vorgewählten oberen Arbeitsgrenzwert ansteigt; und
(4) man läßt Fluid aus dem Wägebeutel in die Fluidströmungseinrichtung einströmen, bis das Gewicht des Fluids in dem Beutel unter den unteren Arbeitsgrenzwert abnimmt.

22. Verfahren nach Anspruch 21,
das ferner den Schritt der automatischen Steuerung der Strömung des Fluids aus dem Behälter oder aus den Behältern (76) zu dem Wägebeutel (80) umfaßt, so daß der Wägebeutel mit Fluid aus dem Behälter oder den Behältern (76) wieder aufgefüllt wird, ohne den Betrieb der Fluidströmungsvorrichtung zu stoppen.

23. Verfahren nach Anspruch 21 oder 22,
welches die Wiederholung der Schritte (1), (2), (3) und (4) für eine Vielzahl von Malen umfaßt.

24. Verfahren nach Anspruch 21 oder 22,
wobei die getrennte Menge des Fluids 100 Gramm bis 800 Gramm beträgt.

25. Verfahren nach Anspruch 23,
wobei jede getrennte Menge des Fluids 100 Gramm bis 200 Gramm beträgt.

26. Verfahren nach einem der Ansprüche 21 bis 25, das ferner folgende Schritte aufweist:
(5) die Alarmeinrichtung wird ausgelöst, um ein Alarmsignal abzugeben, wenn das Gewicht des Fluids in dem Wägebeutel (80) auf einen Wert unter einen zweiten Alarmwert abnimmt, der niedriger ist als der untere Arbeitsgrenzwert; und
(6) man läßt, in Abhängigkeit von dem Alarmsignal, eine zusätzliche getrennte Menge des Fluids aus den oder zumindest einem von den Behältern in den Wägebeutel (80) strömen, so daß das Gewicht des Fluids in dem Wägebeutel (80) über den zweiten Alarmwert erhöht wird.

27. Verfahren zum Betreiben der Anordnung nach Anspruch 18, um zu verhindern, daß Luft in die Fluidströmungsvorrichtung eintritt, wenn die oder zumindest der eine der Fluidbehälter (76) leer sind, wobei das Verfahren folgende Schritte aufweist:
(1) man läßt eine erste getrennte Menge des Fluids aus den oder zumindest einem von den Fluidbehältern (76) in den Wägebeutel (80) strömen;
(2) der Wägebeutel (80) wird gewogen;
(3) der Wägebeutel wird während des Wagens von Strömungsstörungen abgetrennt;
(4) man läßt Fluid aus dem Wägebeutel kontinuierlich in die Fluidströmungsvorrichtung strömen, während die Menge des Fluids in dem Wägebeutel (80) zwischen oberen und unteren Arbeitsgrenzwerten gehalten wird, indem man den Wägebeutel von den oder zumindest dem einen von den Behältern (76) wieder auffüllt.

28. Verfahren nach Anspruch 27,
bei dem aufgrund einer nicht ausreichenden Fluidquelle die Menge des Fluids in dem Wägebeutel (80) unter den unteren Arbeitsgrenzwert abfällt, wobei das Verfahren ferner folgende Schritte aufweist:
(5) es wird ein Alarmsignal erzeugt;
(6) man läßt in Abhängigkeit von dem zweiten Alarmsignal eine zusätzliche getrennte Menge des Fluids aus zumindest der einen zusätzlichen Ersatzfluidquelle in den Wägebeutel strömen, so daß das Gewicht des Fluids in dem Wägebeutel (80) über dem unteren Arbeitsgrenzwert bleibt.

29. Verfahren nach Anspruch 28,
das ferner den Schritt umfaßt, daß gewährleistet wird, daß die Strömung aus dem Wägebeutel (80) ohne Unterbrechung fortgesetzt wird, während der Schritt (5) durchgeführt wird.

30. Verfahren nach Anspruch 28 oder 29,
das ferner den Schrittt umfaßt, daß das Ausströmen aus dem Wägebeutel (80) vorübergehend unterbrochen wird, während der Schritt (5) durchgeführt wird.

31. Verfahren nach einem der Ansprüche 21 bis 30,
das ferner folgende Schritte aufweist:
- die Alarmeinrichtung wird ausgelöst, um ein Alarmsignal auszulösen, wenn das Gewicht des Fluids in dem Wägebeutel unter einen zweiten Alarmwert abnimmt, wobei der zweite Alarmwert niedriger ist als der untere Arbeitsgrenzwert;
- in Abhängigkeit von dem Alarmsignal wird die Strömung aus dem Wägebeutel (80) gesperrt;
- man läßt eine ausreichende zusätzliche getrennte Menge des Fluids von zumindest der einen zusätzlichen Ersatzfluidquelle (76) in den Wägebeutel (80) strömen, um das Gewicht des darin enthaltenen Fluids über den ersten Alarmwert zu erhöhen; und
- das Ausströmen des Fluids aus dem Wägebeutel (80) in die Fluidströmungsvorrichtung wird wieder eingeleitet.

32. Verfahren nach Anspruch 31,
das ferner den Schritt aufweist, daß die Strömung aus dem Wägebeutel (80) in die Fluidströmungsvorrichtung automatisch wieder eingeleitet wird, wenn das Gewicht des Fluids in dem Wägebeutel bis zu einem vorgewählten Wiedereinleitungswert nach dem Absperrschritt ansteigt.

33. Verfahren nach Anspruch 31 oder 32,
wobei jede getrennte Menge des Ersatzfluids 100 Gramm bis 200 Gramm des Fluids beträgt.

34. Verfahren nach Anspruch 33,
wobei der erste höhere Alarmwert 65 Gramm bis 85 Gramm beträgt.

35. Verfahren nach Anspruch 32,
bei dem der zweite Alarmwert unter 15 Gramm liegt und das den Schritt aufweist, daß die Fluidströmung automatisch wieder eingeleitet wird, wenn das Gewicht des Fluids in dem Wägebeutel über den Wiedereinleitungswert ansteigt.

36. Verfahren nach einem der Ansprüche 26 bis 35,
das ferner den Schritt aufweist, daß die Gewichte der getrennten Mengen von Fluid, die in den Wägebeutel strömen, addiert werden, um die Gesamtmenge des Fluids zu berechnen, das von dort in das Strömungssystem geflossen ist.

## Revendications

1. Appareil de monitorage du poids d'un écoulement de fluide hors de conteneurs, ledit appareil comprenant : au moins un conteneur de fluide de remplacement (76) de capacité limitée ; une ligne d'écoulement de fluide d'centrée (82) connectée à une extrémité à la sortie du conteneur et à un sac de pesage (80) à l'autre extrémité ; une ligne d'écoulement de fluide de sortie ayant une entrée et une sortie, l'entrée étant connectée à la sortie du sac de pesage (80) ; et des moyens de pesage (84) disposés pour peser le contenu de fluide dudit sac de pesage (80) ; caractérisé par des moyens d'alarme connectés auxdits moyens de pesage (84) pour déclencher un signal d'alarme quand le poids dudit sac de pesage (80) tombe en dessous d'une valeur d'avertissement présélectionnée ; des moyens (82) pour préconnecter une pluralité de conteneurs de fluide de remplacement (76) à ladite ligne d'écoulement de fluide d'entrée (82) ; et des moyens de pincement (78) sur ladite ligne d'écoulement de fluide d'entrée pour ouvrir ou fermer l'écoulement dans ladite ligne d'écoulement fluide.

2. Appareil selon la revendication 1, comprenant en outre des moyens de commande automatique connectés auxdits moyens de pesage (84) pour commander l'écoulement vers et hors du sac de pesage (80).

3. Appareil selon la revendication 1, dans lequel les moyens de commande comportant des moyens de sécurité en cas de défaillance connectés auxdits moyens de pesage pour fermer l'écoulement hors dudit sac de pesage (80) quand le poids de fluide dans le sac tombe en dessous d'une valeur limite de travail inférieure présélectionnée la valeur d'avertissement étant supérieure à la valeur limite de travail inférieure.

4. Appareil selon la revendication 3, dans lequel les moyens de sécurité en cas de défaillance sont connectés en outre à la pince (78) et ouvrent automatiquement la pince quand le poids de fluide dans le sac de pesage (80) atteint la valeur limite de travail inférieure et ferment automatiquement la pince (78) quand le poids de fluide dans le sac de pesage atteint une valeur limite de travail supérieure.

5. Appareil selon la revendication 3 ou 4, dans lequel les moyens de commande sont agencés pour déclencher l'alarme d'avertissement quand le poids de fluide dans le sac de pesage tombe en dessous d'une seconde valeur d'avertissement, la seconde valeur d'avertissement étant inférieure à la valeur limite de travail inférieure.

6. Appareil selon la revendication 3, 4, ou 5, dans lequel la valeur limite de travail inférieure est de 15 grammes de fluide.

7. Appareil selon l'une quelconque des revendications 3 à 6, dans lequel les moyens de commande sont agencés pour redéclencher l'écoulement hors du sac de pesage (80) après une fermeture de sécurité pour défaillance, quand le fluide dans le sac de pesage (80) dépasse une valeur présélectionée de redéclenchement comprise entre la valeur d'avertissement et la valeur limite de travail inférieure.

8. Appareil selon la revendication 7, dans lequel la valeur présélectionnée de redéclenchement est d'au moins 30 grammes supérieure à la valeur limite de travail inférieure.

9. Appareil selon l'une quelconque des revendications précédentes, dans lequel la ou la première valeur d'avertissement est comprise entre 75 et 85 grammes de fluide.

10. Appareil selon l'une quelconque des revendications précédentes comportant une pompe (72) dans la ligne d'écoulement de fluide de sortie, les moyens de commande étant agencés pour commander la pompe (72) pour retirer du fluide du sac de pesage (80) et engendrer un signal d'erreur quand la pompe (72) a fonctionné pendant un temps et à un débit suffisants pour extraire une quantité présélectionnée de fluide du sac de pesage (80) , mais que le poids de fluide dans le sac de pesage (80) n'a pas baissé d'une valeur correspondante.

11. Appareil selon la revendication 10, dans lequel les moyens de commande sont agencés pour engendrer un signal d'erreur quand la pompe (72) a fonctionné à un débit suffisant pour extraire 150 grammes de fluide du sac de pesage (80).

12. Appareil selon l'une quelconque des revendications précédentes dans lequel une pluralité de conteneurs (76) sont connectés à la ligne d'écoulement fluide d'entrée (82).

13. Appareil selon l'une quelconque des revendications précédentes, dans lequel une chambre d'égouttement (86) est située sur ladite ligne d'écoulement fluide d'entrée (82) pour enlever les bulles des fluides introduite dans le système d'écoulement de fluide.

14. Appareil selon l'une quelconque des revendications précédentes, dans lequel ledit appareil comprend en outre des moyens (90) de support de fluide agencés pour supporter une pluralité de conteneurs de fluide (76) de capacité limitée de manière qu'au moins un desdits conteneurs soit connecté audit sac de pesage (80), par l'intermédiaire de la ligne d'écoulement fluide d'entrée (82).

15. Appareil selon la revendication 14, dans lequel le fluide s'écoule par gravité des conteneurs (76) dans le sac de pesage (80).

16. Appareil selon la revendication 14 ou 15, dans lequel chaque conteneur de remplacement de fluide (76) est attaché à l'entrée dans un piège à gouttes (86) et la sortie du piège à gouttes est attachée à la ligne d'écoulement de fluide d'entrée (82).

17. Appareil selon l'une quelconque des revendications 3 à 8, dans lequel les moyens de commande fonctionnement de manière à monitorer le poids total de fluide de remplacement passé depuis la source d'approvisionnement à la ligne d'écoulement de fluide de sortie.

18. Ensemble de monitorage de la quantité de fluide s'écoulant de conteneurs de fluide de remplacement dans une ligne d'écoulement jetable destinée à la plasmaphérèse, ledit ensemble comprenant un appareil selon l'une quelconque des revendications précédentes, la sortie de la ligne d'écoulement de fluide de sortie étant connectée à un appareil d'écoulement de fluide jetable pour plasmaphérèse afin d'extraire du sang complet d'un sujet, de le séparer et d'en recucillir une première fraction et pour reperfuser le sujet avec un mélange de fluide de remplacement et de la fraction restante de sang.

19. Ensemble comprenant un appareil selon l'une quelconque des revendications 1 à 17, dont la sortie de la ligne d'écoulement de fluide de sortie est connectée à un appareil d'écoulement de fluide.

20. Ensemble selon la revendication 18 ou 19, destiné à un système d'échange de plasma pour séparer le sang reçu d'un sujet en ses constituants et perfuser le sujet avec une premier constituant du sang et un fluide de remplacement, ledit appareil d'écoulement de fluide comprenant : un séparateur (48) pour séparer des premier et second constituants à partir du sang entier : des moyens de connexion (12) pour extraire continûment le sang entier d'un sujet et envoyer ce sang au séparateur (48) ; dés moyens (16, 20, 14, 15) pour introduire de l'anticoagulant dans le sang entier extrait du sujet ; des moyens de connexion (13) pour perfuser continûment le sujet avec le premier constituant du sang et un fluide de remplacement ; des moyens d'écoulement (12, 15, 22, 26, 30) incluant les moyens de connexion d'extraction (12) pour faire écouler le sang entier et l'anticoagulant vers le séparateur (48) et le fluide de remplacement depuis la source (76) de fluide de remplacement et pour faire écouler le premier constituant du sang et fluide de remplacement vers le sujet ; les moyens d'alarme fonctionnant pour déclencher un signal d'avertissement quand la source d'approvisionnement (76) est près d'être vide ; et l'autre desdits appareils comportant des moyens (78) pour délivrer de manière séquentielle des quantités isolées de fluide de remplacement au sac de pesage en vue du pesage séquentiel par les moyens de pesage (84).

21. Procédé pour faire fonctionner l'ensemble de la revendication 19 de manière à empêcher l'air d'entrer dans le système d'écoulement quand le, ou au moins l'un des, conteneurs de fluide de remplacement (76) est vide, ledit procédé comprenant les étapes consistant à :
(1) faire écouler une première quantité isolée de fluide du, ou au moins de l'un des, conteneurs (76) dans le sac de pesage (80) ;
(2) peser le fluide dans le sac de pesage (80) ;
(3) commander l'écoulement de fluide hors du conteneur ou des conteneurs (76), de manière que le fluide s'écoule dans le sac de pesage (80) quand le poids de fluide dans le sac est tombé en dessous d'une valeur limite de travail inférieure présélectionnée et l'écoulement de fluide dans le sac de pesage est arrêté quand le poids dans le sac de pesage dépasse une valeur limite de travail supérieure présélectionnée ; et
(4) faire écouler le fluide du sac de pesage dans l'appareil d'écoulement de fluide jusqu'à ce que le poids de fluide dans la sac tombe en dessous de la limite de travail inférieure.

22. Procédé selon la revendication 21, comprenant en outre l'étape de commande automatique de l'écoulement de fluide hors du ou des conteneurs (76) vers le sac de pesage (80) de façon que le sac de pesage soit rempli de fluide en provenance du ou des conteneurs (76) sans arrêt du fonctionnement de l'appareil d'écoulement de fluide.

23. Procédé selon la revendication 21 ou 22 comportant en outre les étapes de répétition des étapes (1), (2), (3) et (4) plusieurs fois.

24. Procédé selon la revendication 21 ou 22, dans lequel la quantité isolée de fluide est comprise entre 100 et 800 grammes.

25. Procédé selon la revendication 23, dans lequel chaque quantité isolée de fluide est comprise entre 100 et 200 grammes.

26. Procédé selon l'une quelconque des revendications 21 à 25, comportant en outre les étapes de :
(5) déclenchement des moyens d'alarme pour déclencher un signal d'avertissement quand le poids de fluide dans le sac de pesage (80) tombe en dessous d'une seconde valeur d'avertissement qui est inférieure à la valeur limite de travail inférieure ; et
(6) écoulement, en réponse au signal d'avertissement, dans le sac de pesage (80) d'une quantité isolée additionnelle de fluide à partir du ou au moins de l'un des conteneurs de façon que le poids de fluide dans le sac de pesage (80) s'élève au-dessus de la seconde valeur d'avertissement.

27. Procédé pour faire fonctionner l'ensemble selon la revendication 18, de façon à empêcher l'air d'entrer dans l'appareil d'écoulement de fluide quand le ou au moins l'un des conteneurs (76) est vide, le procédé comprenant :
(1) l'écoulement d'une première quantité isolée de fluide du ou au moins de l'un des conteneurs (76) vers le sac de pesage (80) ;
(2) le pesage du sac de pesage (80) ;
(3) l'isolement du sac de pesage des perturbations d'écoulement pendant le pesage ;
(4) l'écoulement continu du fluide depuis le sac de pesage vers l'appareil d'écoulement de fluide tout en maintenant la quantité de fluide dans le sac de pesage (80), entre les valeurs de travail limites supérieure et inférieure par remplissage du sac de pesage à partir du ou au moins de l'un des conteneurs (76).

28. Procédé selon la revendication 27, dans lequel la quantité de fluide dans le sac de pesage (80) tombe en dessous d'une valeur limite de travail inférieure en raison d'une insuffisance de la source de fluide, comportant en outre les étapes de :
(5) génération d'un signal d'avertissement ;
(6) écoulement d'une quantité isolée additionnelle de fluide depuis au moins une source de fluide de remplacement additionnel dans le sac de pesage en réponse au second signal d'alarme de façon que le poids de fluide dans le sac de pesage (80) reste au-dessus de la valeur limite de travail inférieure.

29. Procédé selon la revendication 28, comportant en outre l'étape de vérification que l'écoulement hors du sac de pesage (80) continue de façon ininterrompue pendant l'accomplissement de l'étape (5).

30. Procédé selon la revendication 28 ou 29, comportant en outre l'étape d'interruption temporaire de l'écoulement hors du sac de pesage (80) pendant l'accomplissement de l'étape (5).

31. Procédé selon l'une quelconque des revendications 21 à 30, comportant en outre les étapes de :
déclenchement des moyens d'alarme pour déclencher un signal d'avertissement quand le poids de fluide dans le sac de pesage tombe en dessous d'une seconde valeur d'avertissement, la seconde valeur d'avertissement étant inférieure à la valeur limite de travail inférieure ;
fermeture de l'écoulement hors du sac de pesage (80) en réponse au signal d'avertissement ;
écoulement de suffisamment d'une quantité isolée additionnelle de fluide en provenance d'au moins une source de fluide de remplacement additionnelle (76) dans le sac de pesage (80) pour élever le poids de fluide qui s'y trouve au-dessus de la première valeur d'avertissement ; et
redémarrage de l'écoulement à partir dudit sac de pesage (80) dans ledit appreil d'écoulement de fluide.

32. Procédé selon la revendication 31, comportant en outre l'étape de redémarrage automatique de l'écoulement hors du sac de pesage (80) dans l'appareil d'écoulement de liquide quand le poids de fluide dans le sac de pesage atteint une valeur présélectionnée de redémarrage après l'étape de fermeture.

33. Procédé selon la revendication 31 ou 32, dans lequel chaque quantité isolée de fluide de remplacement comprend entre 100 et 200 grammes de fluide.

34. Procédé selon la revendication 33, dans lequel la première valeur supérieure d'avertissement est comprisé entre 65 et 85 grammes.

35. Procédé selon la revendication 32, dans lequel la seconde valeur d'avertissement est inférieure à 15 grammes, et comportant l'étape de redémarrage automatique de l'écoulement de fluide quand le poids de fluide dans le sac de pesage dépasse la valeur de redémarrage.

36. Procédé selon l'une quelconque des revendications 26 à 35, comportant en outre l'étape d'addition des poids des quantités isolées de fluide écoulées dans le sac de pesage pour calculer la quantité totale de fluide qui s'en est écoulée vers le système d'écoulement.
